# EUROPEAN PATENT APPLICATION

(11) **EP 4 458 329 A1**
(43) Date of publication of application: **06.11.2024**
(21) Application number: 22914642.8
(22) Date of filing: 26.12.2022
(51) Int. Cl.: A61F 2/95

(54) **SHEATH CORE ASSEMBLY, FABRICATION METHOD FOR SHEATH CORE ASSEMBLY, AND DELIVERY DEVICE**

(30) Priority: 30.12.2021 CN 202111668219
(71) Applicant: Lifetech Scientific (Shenzhen) Co., Ltd., Shenzhen, Guangdong 518063 (CN)
(72) Inventor: TANG, Jiangfeng, Shenzhen, Guangdong 518063 (CN); XIAO, Benhao, Shenzhen, Guangdong 518063 (CN)
(74) Representative: Michalski Hüttermann & Partner Patentanwälte mbB
(86) International application number: PCT/CN2022/141845
(87) International publication number: WO 2023/125388

(57) **Abstract**

A sheath core assembly, a method for manufacturing a sheath core assembly, and a delivery device are provided. The sheath core assembly includes a sheath core (11), and a tip (14) and a connector which are provided at a distal end of the sheath core (11). The tip (14) includes a first connecting section (141) and a guiding section (142) coaxially connected to a distal end of the first connecting section (141). The guiding section (142) is tapered and has an outer diameter gradually decreasing from a proximal end to a distal end thereof. The connector is connected between the sheath core (11) and the tip (14). A proximal end of the connector extends out from a proximal face of the tip (14), and a distal end of the connector extends towards a distal end of the tip (14) and forms an extending section (122). The extending section (122) is embedded in the guiding section (142). The extending section (122) is tapered and has an outer diameter gradually decreasing from a proximal end to a distal end thereof. The problems of difficult cleaning and breakage of a tip (4') of an existing delivery system caused by bubbling in injection
molding of the tip (4') can be solved.

## Description

### Technical Field

The present disclosure relates to the technical field of interventional medical instruments, and more particularly to a sheath core assembly, a method for manufacturing a sheath core assembly, and a delivery device.

### Background Art

Minimally invasive stent-grafted vascular repair is widely used in the treatment of aortic vascular diseases because of small wound, quick recovery, and good immediate effect. During clinical implantation, a covered stent is preloaded into a sheath catheter of a delivery system and then passes through the vascular lumen of a human body. After being delivered to a lesion position by the delivery system, the stent is released from the sheath catheter. The stent isolates blood flow from vascular lesion, so as to achieve the purpose of treatment.

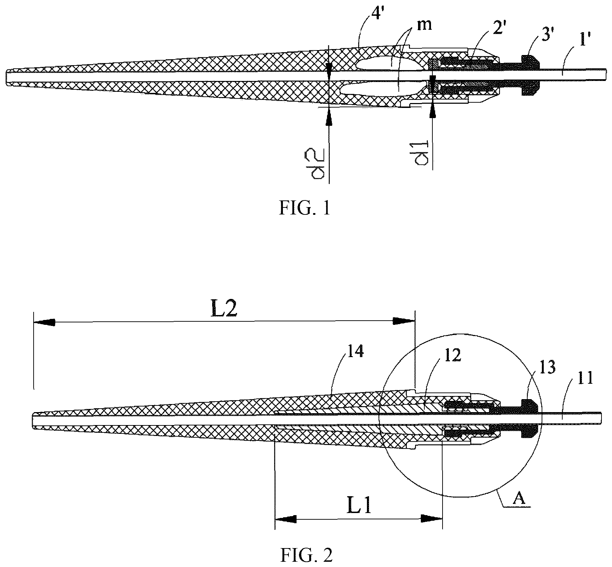

Since the delivery system for the stent needs to enter the blood vessel, the stent is delivered to a predetermined position. In order to advance the delivery system smoothly in the blood vessel, a tip having a tapered structure is usually designed at a front end of the delivery system. The tip is generally made of a soft polymer material by injection molding. In order to ensure the connection strength between the tip and a sheath core of a delivery device made of a stainless steel tube, the tip is connected to the sheath core through a plurality of connectors made of different materials. The structure of the tip is shown in FIG. 1. A distal end of the delivery system includes a sheath core 1' made of a stainless steel tube, an inverted buckle 2', an inverted hook 3', and a tip 4'. The inverted buckle 2' is made of a metal material and is connected to the stainless steel tube by welding to prevent the tip 4' from falling off. The inverted hook 3' is made of a polymer material, which is harder than that of the tip 4', and is fixedly connected to the inverted buckle 2'. The design of the inverted hook 3' may ensure the smooth release of a bare stent. During injection molding of the tip 4', due to the large difference in wall thickness at different parts, such as d1 and d2 shown in the figure, it is easy to form a large temperature difference during injection cooling. The cooling is uneven, and shrinking bubbles are easily formed at a position with a large wall thickness. After bubbles are formed in an inner cavity of the tip 4', the structural strength of the tip is reduced, and there is a risk of fracture. At the same time, once entering the bubbles, water is difficult to remove and dry, which is easy to breed bacteria and increases the difficulty of sterilization. Once inadequately sterilized instruments enter human bodies, patients are vulnerable to infection, and the lives of the patients are even threatened.

### Summary of the Invention

In view of this, the present disclosure provides a sheath core assembly, a method for manufacturing a sheath core assembly, and a delivery device, intended to solve the problems of difficult cleaning and breakage of a tip of an existing delivery system caused by bubbling in injection molding of the tip.

To achieve the above object, the present disclosure adopts a technical solution as follows.

The present disclosure provides a sheath core assembly, including a sheath core, and a tip and a connector which are provided at a distal end of the sheath core. The tip includes a first connecting section and a guiding section coaxially connected to a distal end of the first connecting section. The guiding section is tapered and has an outer diameter gradually decreasing from a proximal end to a distal end thereof. The connector is connected between the sheath core and the tip. A proximal end of the connector extends out from a proximal face of the tip, and a distal end of the connector extends towards a distal end of the tip and forms an extending section. The extending section is embedded in the guiding section. The extending section is tapered and has an outer diameter gradually decreasing from a proximal end to a distal end thereof.

In one embodiment, a taper difference between the guiding section and the extending section is ±5°.

In one embodiment, the connector includes an inverted buckle and an inverted hook. The inverted buckle is coaxially sleeved to the distal end of the sheath core and relatively fixed to the sheath core. The inverted hook includes a second connecting section and a blocking section coaxially connected to a proximal end of the second connecting section, and the second connecting section is relatively fixed to the inverted buckle. The tip wraps outer peripheries of the inverted buckle and the second connecting section, whereby only the blocking section extends out of the proximal face of the tip.

In one embodiment, the inverted buckle includes a third connecting section and the extending section coaxially connected to a distal end of the third connecting section. The third connecting section is relatively fixed to the sheath core after the inverted buckle is coaxially sleeved to the distal end of the sheath core. The second connecting section is sleeved to the third connecting section and relatively fixed to the third connecting section after the inverted hook is sleeved to the distal end of the sheath core.

In one embodiment, the third connecting section includes a first welding section and a sleeving section coaxially connected to a proximal end of the first welding section. An outer diameter of the sleeving section is greater than that of the first welding section. An outer periphery of the sleeving section is provided with an external thread, and an inner periphery of the second connecting section is provided with an internal thread. The first welding section is relatively fixed to the sheath core after the inverted buckle is coaxially sleeved to the distal end of the sheath core. The second connecting section is sleeved to the sleeving section and threaded to the sleeving section after the inverted hook is sleeved to the distal end of the sheath core.

In one embodiment, an axial length of the extending section is less than or equal to one third of an axial length of the guiding section.

In one embodiment, an anti-revolving groove is recessed in a surface of the extending section.

In one embodiment, the inverted hook further includes the extending section coaxially connected to a distal end of the second connecting section, and the inverted hook wraps the distal end of the sheath core and the outer periphery of the inverted buckle, whereby the inverted hook is relatively fixed to the inverted buckle.

In one embodiment, the inverted buckle includes a second welding section sleeved to the sheath core and relatively fixed to the sheath core and a limiting arm radially extending from a proximal end of the second welding section. The limiting arm is embedded in the second connecting section of the inverted hook when the inverted hook wraps the distal end of the sheath core and the outer periphery of the inverted buckle, whereby the inverted hook is relatively fixed to the inverted buckle.

In one embodiment, the second connecting section includes a first sleeve and a second sleeve connected to each other. An outer diameter of the first sleeve is greater than that of the second sleeve. The first sleeve is provided with a clamping groove in which the limiting arm is embedded.

In one embodiment, at least two limiting arms are circumferentially provided at intervals at the proximal end of the second welding section. At least two clamping grooves are provided on the first sleeve. One limiting arm is embedded in one clamping groove.

In one embodiment, an anti-rotation groove is further recessed in a surface of the first sleeve, and/or an anti-rotation block is provided on the second sleeve.

In one embodiment, an axial length of the extending section is less than or equal to one half of an axial length of the guiding section.

In one embodiment, the inverted buckle is a metal member, and/or the inverted hook is integrally formed by injection molding.

The present disclosure adopts another technical solution.

The present disclosure provides a method for manufacturing the sheath core assembly as described above. The method includes:
providing a sheath core and an inverted buckle;
coaxially sleeving the inverted buckle to a distal end of the sheath core, and relatively fixing the inverted buckle to the sheath core;
forming an inverted hook at the distal end of the sheath core and an outer periphery of the inverted buckle by injection molding, where the formed inverted hook includes a second connecting section relatively fixed to the inverted buckle, and a blocking section and an extending section coaxially connected to a proximal end and a distal end of the second connecting section, and the extending section is tapered and has an outer diameter gradually decreasing from a proximal end to a distal end thereof; and
then forming a tip at the outer peripheries of the inverted buckle and the second connecting section by injection molding, where the tip includes a first connecting section and a guiding section coaxially connected to a distal end of the first connecting section, and the guiding section is tapered and has an outer diameter gradually decreasing from a proximal end to a distal end thereof.

The present disclosure adopts yet another technical solution.

The present disclosure provides a delivery device, including the sheath core assembly as described above.

The present disclosure provides a sheath core assembly and a delivery device including the sheath core assembly. A distal end of a connector extends, and the shape of an extending section is adapted to a tapered guiding section of a tip as much as possible, whereby the wall thickness of the tip formed by injection molding is as even as possible, thus avoiding a large difference of the wall thickness. At the same time, the flow of injection liquid can be guided during injection molding, which is more beneficial to the molding of the tip and reduces the generation of cooling shrinkage bubbles during injection molding under the same technological conditions. In addition, the connector having the extending section may also improve the connection strength between the sheath core and the tip, and stability and reliability are taken into account. The present disclosure provides a method for manufacturing a sheath core assembly. The wall thickness of each injection molding is even, thus greatly reducing the risk of bubbles generated by injection molding. In addition, the structure of an inverted buckle is greatly simplified, the machining difficulty of the inverted buckle is reduced, and the cost of parts is reduced. More importantly, through step-by-step integrated injection molding, the mounting process of an inverted hook is omitted, thus simplifying the production process and improving the production efficiency.

### Brief Description of the Drawings

FIG. 1 is a schematic partial cross-sectional view of an existing sheath core assembly;
FIG. 2 is a schematic partial cross-sectional view of a sheath core assembly according to Embodiment 1 of the present disclosure;
FIG. 3 is a schematic enlarged view of part A in FIG. 2;
FIG. 4 is a schematic cross-sectional view of an inverted buckle in a sheath core assembly according to Embodiment 1 of the present disclosure;
FIG. 5 is a schematic cross-sectional view of an inverted hook in a sheath core assembly according to Embodiment 1 of the present disclosure;
FIG. 6 is a schematic cross-sectional view of a tip in a sheath core assembly according to Embodiment 1 of the present disclosure;
FIG. 7 is a schematic partial cross-sectional view of a sheath core assembly according to Embodiment 2 of the present disclosure;
FIG. 8 is a schematic enlarged view of part B in FIG. 2;
FIG. 9 is a schematic cross-sectional view of an inverted buckle in a sheath core assembly according to Embodiment 2 of the present disclosure;
FIG. 10 is a schematic top view of an inverted buckle in a sheath core assembly according to Embodiment 2 of the present disclosure;
FIG. 11 is a schematic cross-sectional view of an inverted hook in a sheath core assembly according to Embodiment 2 of the present disclosure;
FIG. 12 is a schematic view depicting a three-dimensional structure of an inverted hook in a sheath core assembly according to Embodiment 2 of the present disclosure;
FIG. 13 is a schematic cross-sectional view of a tip in a sheath core assembly according to Embodiment 2 of the present disclosure; and
FIG. 14(a) to FIG. 14(d) are schematic views depicting manufacturing processes of a sheath core assembly according to Embodiment 2 of the present disclosure.

### Detailed Description of the Invention

In order that the objects, technical solutions, and advantages of the present disclosure will become more apparent, exemplary implementations of the present disclosure will be described in detail below with reference to the accompanying drawings. While the drawings show exemplary implementations of the present disclosure, it should be understood that the present disclosure may be embodied in various forms and should not be limited by the implementations set forth herein. Rather, these implementations are provided so that the present disclosure will be thoroughly understood, and the scope of the present disclosure will be fully conveyed to those skilled in the art.

It should be understood that the terms used herein are for the purpose of describing particular example implementations only and are not intended to be limiting. As used herein, the singular forms "a/an", "one", and "the" are intended to include the plural forms as well, unless the context clearly indicates otherwise. The terms "comprising", "including", "containing", and "having" are inclusive and therefore specify the presence of stated features, steps, operations, elements, and/or components, but do not preclude the presence or addition of one or more other features, steps, operations, elements, components, and/or combinations thereof. The method steps, processes, and operations described herein are not to be construed as necessarily requiring to be performed in the particular order described or illustrated, unless an order of performance is explicitly stated. It should also be understood that additional or alternative steps may be used.

Although the terms first, second, third, and the like may be used herein to describe various elements, components, regions, layers, and/or sections, these elements, components, regions, layers, and/or sections should not be limited by these terms. These terms may be used only to distinguish one element, component, region, layer, or section from another region, layer, or section. The use of terms such as "first", "second", and other numerical terms herein does not imply a sequence or order unless the context clearly dictates otherwise. Therefore, a first element, component, region, layer, or section discussed below may be referred to as a second element, component, region, layer, or section without departing from the teachings of example implementations.

For ease of description, spatially relative terms, such as "inner", "outer", "inside", "outside", "underneath", "below", "over", and "above", may be used herein to describe the relationship between one element or feature and another element or feature as illustrated in the figures. Such spatially relative terms are intended to encompass different orientations of a device in use or operation in addition to the orientations depicted in the figures. For example, if the device in the figures is turned over, elements described as "underneath" or "below" other elements or features would then be oriented "over" or "above" the other elements or features. Therefore, the example term "below" may include orientations: above and below. The device may be otherwise oriented (rotated by 90 degrees or in other directions) and is interpreted accordingly by spatially relative descriptors used herein.

It should be additionally noted that in the field of interventional medical instruments, an end of a medical instrument implanted in a human or animal body or a delivery system for delivering the medical instrument, which is closer to an operator, is generally referred to as a "proximal end", an end which is further away from the operator is referred to as a "distal end", and the "proximal end" and "distal end" of any component of the medical instrument or the delivery system are defined in accordance with this principle. An "axial direction" generally refers to a lengthwise direction of the medical instrument when being delivered, and a "radial direction" generally refers to a direction of the medical instrument perpendicular to the "axial direction" thereof, and the "axial direction" and "radial direction" of any component of the medical instrument are defined in accordance with this principle.

Referring to FIG. 1, a sheath core assembly adopted in an existing delivery system generally includes a sheath core 1', an inverted buckle 2', an inverted hook 3', and a tip 4'. The sheath core 1' and the inverted buckle 2' are metal members machined from metal materials. The inverted hook 3' is an injection molded member formed by a separate injection molding process. The injection-molded inverted hook 3' is mounted at a distal end of the sheath core 1' to ensure smooth release of a bare stent during stent release. Since the sheath core 1' is generally thin in wall, it is not easy to mount the inverted hook 3' to the distal end of the sheath core 1'. Therefore, the sheath core 1' and the inverted hook 3' are connected by the inverted buckle 2'. Therefore, an existing mounting and manufacturing method includes: sleeving the machined metal member namely the inverted buckle 2' to the distal end of the stainless steel sheath core 1', and welding the inverted buckle 2' to the sheath core 1', so as to be relatively fixed to each other, where an outer peripheral wall of a proximal end of the machined inverted buckle 2' is provided with an external thread; then, sleeving the injection-molded inverted hook 3' to the distal end of the sheath core 1', providing an internal thread on an inner peripheral wall of a distal end of the inverted hook 3', and sleeving the distal end of the inverted hook 3' to the outer periphery of the proximal end of the inverted buckle 2' so that the internal thread of the inverted hook is connected to the external thread of the inverted buckle 2', whereby the inverted hook 3' is connected to the inverted buckle 2'; and finally, forming the tip 4' at the outer peripheries of the distal ends of the inverted buckle 2' and inverted hook 3' by injection molding, so as to form the sheath core assembly.

Since a proximal end of the tip 4' in the existing sheath core assembly needs to form a tapered distal end to play a guiding role and also needs to abut against a sheath catheter sleeved on the outer periphery of the sheath core 1', there are usually steps on the injection-molded tip 4', and unequal radial dimensions of the outer periphery contours of the inverted buckle 2' and the inverted hook 3' lead to a large difference in wall thickness at different parts of the tip 4' during injection molding, whereby bubbles m are easily formed in an inner cavity of the tip 4' after injection molding.

In view of this, the present disclosure provides a sheath core assembly. The sheath core assembly includes a sheath core, and a tip and a connector which are provided at a distal end of the sheath core. The connector is configured to connect the tip to the sheath core. A proximal end of the connector extends out from a proximal face of the tip to ensure smooth release of a bare stent, and a distal end of the connector extends towards a distal end of the tip and forms an extending section. The extending section is embedded in the guiding section.

Specifically, the sheath core is a tubular metal member, and, exemplarily, a stainless steel tube. The tip includes a first connecting section and a guiding section coaxially connected to a distal end of the first connecting section. The guiding section is tapered and has an outer diameter gradually decreasing from a proximal end to a distal end thereof. A maximum outer diameter of the guiding section is greater than an outer diameter of the first connecting section located at the proximal end of the tip, whereby a joint of the guiding section and the first connecting section forms a step, and a proximal face of the guiding section abuts against a distal face of a sheath catheter when the sheath catheter is sleeved on an outer periphery of the sheath core. Preferably, the thickness of the step is approximately equal to the wall thickness of the sheath catheter, whereby an outer periphery of the sheath catheter may be flush with a maximum outer periphery of the tip when the sheath catheter is sleeved on the outer periphery of the sheath core and the distal face of the sheath catheter abuts against the proximal face of the guiding section, thus avoiding scratching a blood vessel wall during pushing. The tip is formed on an outer periphery of the connector by injection molding after the connector is fixed to the distal end of the sheath core, whereby the tip is fixed to the distal end of the sheath core. The connector is located between the sheath core and the tip for connecting the sheath core to the tip. The proximal end of the connector extends out from the proximal face of the tip to ensure smooth release of the bare stent, and the distal end of the connector extends towards the distal end of the tip and forms the extending section. The extending section is embedded in the guiding section. The extending section is tapered and has an outer diameter gradually decreasing from a proximal end to a distal end thereof.

In the present disclosure, the distal end of the connector extends, and the shape of the extending section is adapted to the tapered guiding section of the tip as much as possible, whereby the wall thickness of the tip formed by injection molding is as even as possible, thus avoiding a large difference of the wall thickness. At the same time, the flow of injection liquid can be guided during injection molding, which is more beneficial to the molding of the tip and reduces the generation of cooling shrinkage bubbles during injection molding under the same technological conditions. In addition, the connector having the extending section may also improve the connection strength between the sheath core and the tip, and ensure the reliability of connection. Preferably, in order to further ensure the evenness of the wall thickness and the fluidity of glue injection liquid, a taper difference between the guiding section and the extending section is ±5°. Preferably, the taper of the guiding section is equal to that of the extending section, so as to further reduce the generation of bubbles. It should be noted that the tapered tip of the present disclosure is of a non-sharp conical shape.

Exemplarily, the connector of the present disclosure also includes an inverted buckle and an inverted hook based on the consideration of the original design intention of the existing inverted buckle and inverted hook. However, the inverted buckle and/or inverted hook of the present disclosure differ from the existing inverted buckle and inverted hook in that a distal end of the inverted buckle and/or inverted hook of the present disclosure extends towards the distal end of the tip and forms an extending section embedded in the guiding section of the tip. That is, the extending section of the connector may be formed at the distal end of the inverted buckle or at the distal end of the inverted hook. Exemplarily, the sheath core of the present disclosure is a stainless steel tube, the inverted buckle is a metal member machined from a metal material, and the inverted hook is integrally formed by injection molding. The machined inverted buckle is coaxially sleeved to the distal end of the sheath core and relatively fixed to the sheath core. The inverted hook includes a second connecting section and a blocking section coaxially connected to a proximal end of the second connecting section, and the second connecting section is relatively fixed to the inverted buckle. The tip wraps outer peripheries of the inverted buckle and the second connecting section, whereby only the blocking section extends out of the proximal face of the tip to ensure smooth release of a bare stent during stent release. This embodiment uses the existing mode of connecting via the inverted buckle and the inverted hook, whereby the problem of bubbles can be skillfully solved without making big changes to the existing process or structure, the cost is greatly saved, and the stability and reliability of the connection are taken into account at the same time.

Based on the above-mentioned sheath core assembly, the present disclosure also provides a delivery device including the sheath core assembly.

### Embodiment 1

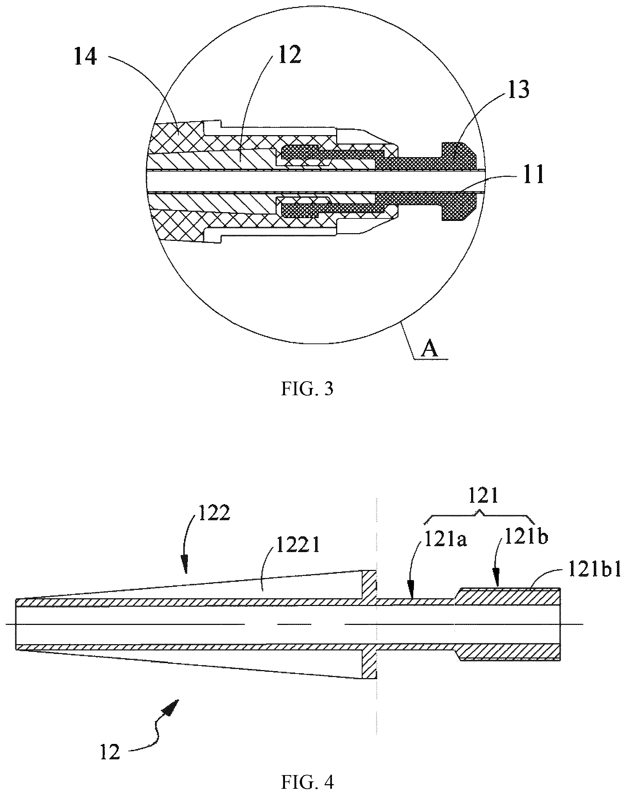

Referring to FIG. 2 and FIG. 3, on the basis of the aforementioned sheath core assembly, the present embodiment exemplarily provides a detailed implementation of the sheath core assembly. The sheath core assembly of this embodiment includes a sheath core 11, and a tip 14 and a connector which are provided at a distal end of the sheath core 11. The connector includes an inverted buckle 12 and an inverted hook 13. An extending section of the connector embedded in the tip 14 is formed at a distal end of the inverted buckle 12. This embodiment mainly describes the structures of the inverted buckle 12 and the inverted hook 13 of the connector in detail and describes the structures of the sheath core 11 and the tip 14 in the sheath core assembly with reference to the foregoing.

Specifically, as shown in FIG. 2 to FIG. 4, the inverted buckle 12 includes a third connecting section 121 and an extending section 122 coaxially connected to a distal end of the third connecting section 121. The extending section 122 extends towards a distal end of the tip 14, and the extending section 122 is embedded in a guiding section of the tip 14. The extending section 122 is in the shape of a taper having an outer diameter gradually decreasing from a proximal end to a distal end thereof. The third connecting section 121 is configured to connect the sheath core 11 to the inverted hook 13. As an implementation of the third connecting section 121 implementing the connection between the sheath core 11 and the inverted hook 13, the third connecting section 121 includes a first welding section 121a and a sleeving section 121b coaxially connected to a proximal end of the first welding section 121a. At this moment, the extending section 122, the first welding section 121a, and the sleeving section 121b are sequentially connected from the distal end to the proximal end to form the inverted buckle 12. Since the inverted buckle 12 is sleeved on the sheath core and then welded to the sheath core 11, the wall thickness of the first welding section 121a is smaller, that is, an outer diameter of the first welding section 121a is smaller for convenience of welding. While the extending section 122 needs to occupy as much space as possible as the tip 14 during injection molding in an initial position, a maximum outer diameter of the extending section 122 is much larger than the outer diameter of the first welding section 121a. The sleeving section 121b is configured to be connected to the inverted hook 13, and an external thread 121b1 is required on an outer wall of the sleeving section 121b. Therefore, the wall thickness of the sleeving section 121b is slightly greater than that of the first welding section 121a. That is, an outer diameter of the sleeving section 121b is slightly greater than that of the first welding section 121a, whereby the external thread 121b1 for connecting the inverted hook 13 may be provided on an outer periphery of the sleeving section 121b. The inverted buckle 12 of this embodiment is a metal member machined from a metal material. In the manufacturing process, the machined inverted buckle 12 is sleeved to the distal end of the stainless steel sheath core 11, and the first welding section 121a of the inverted buckle 12 is welded to the sheath core 11, whereby the inverted buckle 12 and the sheath core 11 are relatively fixed. It should be noted that the manner in which the first welding section 121a is welded is not limited to the fixing manner of this embodiment and the fixing may be achieved by another fixing manner.

Referring to FIG. 2 to FIG. 4, since the tip 14 wraps an outer periphery of the extending section 122 of the inverted buckle 12 by injection molding, an anti-revolving groove 1221 is recessed in a surface of the extending section 122 in other implementations in order to further enhance the connection strength between the tip 14 and the extending section 122. During injection molding of the tip 14, glue injection liquid will enter the anti-revolving groove 1221 recessed in the surface of the extending section 122, thus embedding into the anti-revolving groove 1221 to form a circumferential limit. Preferably, at least two anti-revolving grooves 1221 are provided at intervals in a circumferential direction on the surface of the extending section 122 to enhance the connection strength and the limit strength.

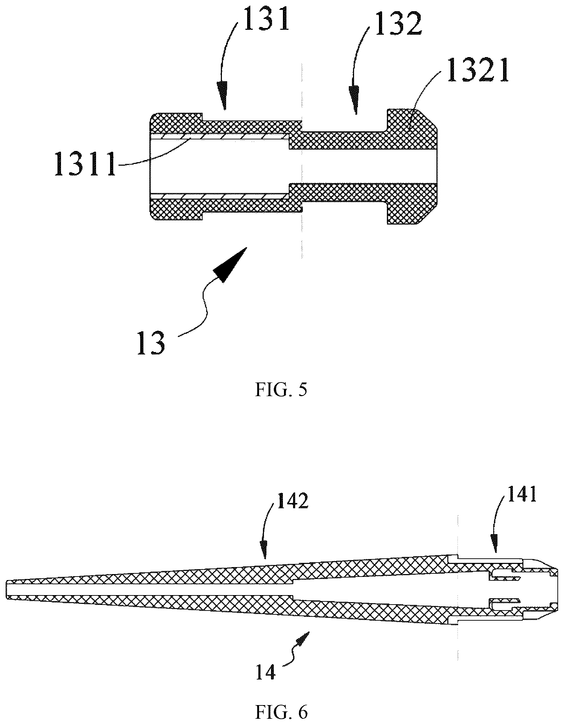

As shown in FIG. 2, FIG. 3, and FIG. 5, the inverted hook 13 includes a second connecting section 131 and a blocking section 132 coaxially connected to a proximal end of the second connecting section 131. An inner periphery of the second connecting section 131 is provided with an internal thread 1311. After the inverted buckle 12 is coaxially sleeved to the distal end of the sheath core 11, the first welding section 121a is relatively fixed to the sheath core 11. After the inverted hook 13 is sleeved to the distal end of the sheath core 11, the second connecting section 131 is sleeved to the sleeving section 121b of the inverted buckle 12 and is threaded to the external thread 121b1 on the sleeving section 121b, whereby the inverted hook 13 and the inverted buckle 12 are relatively fixed. Exemplarily, a proximal tail end of the blocking section 132 is vertically provided with a circular blocking table 1321, and an outer peripheral edge of a proximal end of the blocking table 1321 is rounded to avoid scratching and facilitate the release of a bare stent.

Referring to FIG. 2, the sheath core provided with the inverted buckle 12 and the inverted hook 13 is finally placed in an injection molding machine to injection-mold the tip 14, the injection-molded tip 14 wraps the outer peripheries of the inverted buckle 12 and the second connecting section 131, the blocking section 132 of the inverted hook 13 extends out of a proximal face of the tip 14, and the blocking table is configured to ensure smooth release of a bare stent during stent release.

Referring to FIG. 2, FIG. 3, and FIG. 6, the tip 14 includes a first connecting section 141 and a guiding section 142 coaxially connected to a distal end of the first connecting section 141. The guiding section 142 is in the shape of a taper having an outer diameter gradually decreasing from a proximal end to a distal end thereof. A maximum outer diameter of the guiding section 142 is greater than an outer diameter of the first connecting section 141 located at a proximal end of the guiding section, whereby a joint of the guiding section 142 and the first connecting section 141 forms a step. A proximal face of the guiding section 142 abuts against a distal face of a sheath catheter when the sheath catheter is sleeved on the outer periphery of the sheath core 11. Preferably, the thickness of the step is approximately equal to the wall thickness of the sheath catheter, whereby an outer periphery of the sheath catheter may be flush with a maximum outer periphery of the tip 14 when the sheath catheter is sleeved on the outer periphery of the sheath core 11 and the distal face of the sheath catheter abuts against the proximal face of the guiding section 142, thus avoiding scratching a blood vessel wall during pushing.

Further, referring to FIG. 2, since the inverted buckle 12 is made of metal and has a hard texture and the tip 14 requires a soft front end and good bending performance when being pushed in the blood vessel, a length L1 of the tapered extending section 122 of the inverted buckle 12 is required to be not too large. If the length of the tapered extending section 122 of the inverted buckle 12 is too large, the front end of the tip 14 is easy to harden, thus affecting the pushing performance of the tip 14 in the blood vessel. In addition, if the length of the tapered extending section 122 of the inverted buckle 12 is too small, the taper change will become larger, and as the wall thickness of the tip 14 is more uneven, the risk of bubble generation during injection molding is higher. Therefore, it is necessary to control the tapered extending section 122 of the inverted buckle 12 in a reasonable range. Preferably, an axial length L1 of the extending section 122 is less than or equal to one third of an axial length L2 of the guiding section. An axial length L1' of the extending section 233 crosses at least the step formed by the first connecting section and the guiding section of the tip. That is, the extending section 233 is at least partially embedded in the guiding section of the tip. This length avoids the generation of bubbles as much as possible, while ensuring the bending and flexibility properties of the tip 14.

In this embodiment, the front end of the inverted buckle 12 is designed to be tapered, the taper value is adapted to the taper of the tip 14, and the taper fills a position with a large wall thickness of the tip 14. This taper design achieves the even overall wall thickness of the tip 14. At the same time, the flow of glue injection liquid can be guided during injection molding, which is more beneficial to the molding of the tip 14 and reduces the generation of cooling shrinkage bubbles during injection molding under the same technological conditions. In addition, the anti-revolving groove 1221 is designed on the taper of the inverted buckle 12 to prevent circumferential rotation and enhance the connection strength.

### Embodiment 2

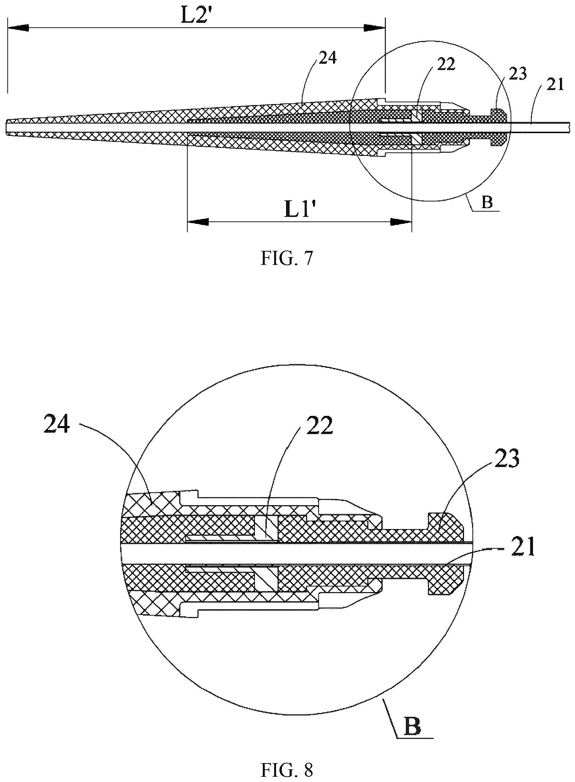

Referring to FIG. 7 and FIG. 8, on the basis of the aforementioned sheath core assembly, the present embodiment exemplarily provides another detailed implementation of the sheath core assembly. The sheath core assembly of this embodiment includes a sheath core 21, and a tip 24 and a connector which are provided at a distal end of the sheath core 21. The connector includes an inverted buckle 22 and an inverted hook 23. An extending section 233 of the connector embedded in the tip 24 is formed at a distal end of the inverted hook 23. This embodiment mainly describes the structures of the inverted buckle 22 and the inverted hook 23 of the connector in detail and describes the structures of the sheath core 21 and the tip 24 in the sheath core assembly with reference to the foregoing.

Specifically, as shown in FIG. 7 to FIG. 9, the inverted buckle 22 is coaxially sleeved to a distal end of the sheath core 21 and is relatively fixed to the sheath core 21. Exemplarily, as an implementation of the inverted buckle 22, the inverted buckle 22 includes a second welding section 221 sleeved to the sheath core 21 and relatively fixed to the sheath core 21 and a limiting arm 222 radially extending from a proximal end of the second welding section 221. The limiting arm 222 is embedded in the second connecting section 231 of the inverted hook 23 when the inverted hook 23 wraps the distal end of the sheath core 21 and an outer periphery of the inverted buckle 22 by injection molding, whereby the inverted hook 23 and the inverted buckle 22 are relatively fixed.

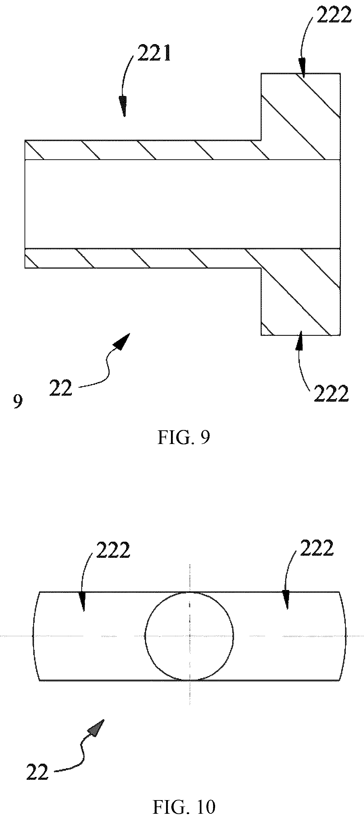

As shown in FIG. 7 to FIG. 9, the second welding section 221 is a tube body structure, and the limiting arm 222 is vertically connected to an outer periphery of a proximal end of the tube body. After the inverted buckle 22 is welded to the distal end of the stainless steel sheath core 21, the end of the inverted buckle 22 provided with the limiting arm 222 is proximal, and the end provided with the second welding section 221 is distal, thus not only enhancing the connection strength between the inverted hook 23 and the inverted buckle 22, but also preventing the inverted hook 23 from breaking at the distal end of the stainless steel sheath core 21. Preferably, referring to FIG. 9 and FIG. 10, in order to ensure the stability of the connection, at least two limiting arms 222 are circumferential provided at intervals at the proximal end of the second welding section 221, at least two clamping grooves 231a1 are provided on a first sleeve 231a, and one limiting arm 222 is embedded in one clamping groove 231a1 to enhance the connection strength and the limiting strength. Compared with the existing inverted buckle 22, the inverted buckle 22 of this embodiment has a simpler structure, and since the inverted buckle 22 and the inverted hook 23 are connected by injection molding wrapping, there is no need to provide a thread structure on the outer periphery of the inverted buckle 22, thus greatly reducing the machining difficulty of the inverted buckle 22 and reducing the cost of parts.

As shown in FIG. 7, FIG. 8, and FIG. 11, the inverted hook 23 includes a second connecting section 231, a blocking section 232 coaxially connected to a proximal end of the second connecting section 231, and an extending section 233 coaxially connected to a distal end of the second connecting section 231. That is to say, the extending section 233 of the aforementioned connector is formed at the distal end of the inverted hook 23. The inverted hook 23 wraps the distal end of the sheath core 21 and the outer periphery of the inverted buckle 22, whereby the inverted hook 23 and the inverted buckle 22 are relatively fixed. In this embodiment, since the inverted hook 23 is connected by wrapping the distal end of the sheath core 21 and the outer periphery of the inverted buckle 22 through injection molding, there is no need to provide a thread structure on the inner periphery of the inverted hook 23, and the mounting process of the inverted hook 23 is omitted, thus simplifying the production process and improving the production efficiency. Exemplarily, a proximal tail end of the blocking section 232 is vertically provided with a circular blocking table 2321, and an outer peripheral edge of a proximal end of the blocking table 2321 is rounded to avoid scratching and facilitate the release of a bare stent.

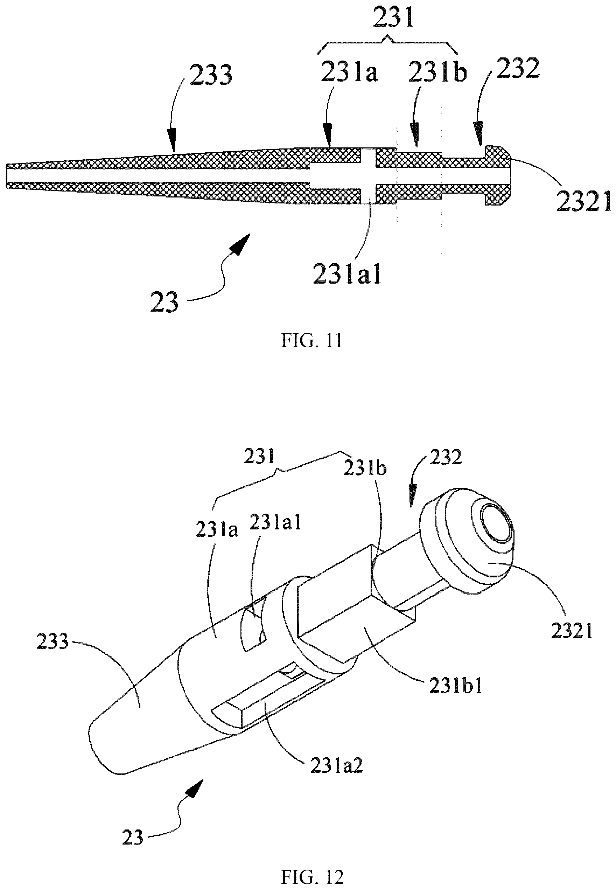

Exemplarily, as shown in FIG. 11 and FIG. 12, the second connecting section 231 includes a first sleeve 231a and a second sleeve 231b connected to each other. An outer diameter of the first sleeve 231a is greater than that of the second sleeve 231b. The first sleeve 231a is provided with a clamping groove 231a1 in which the limiting arm 222 is embedded. The extending section 233 is connected to a distal end of the first sleeve 231a. Preferably, a maximum outer diameter of the extending section 233 is equal to an outer diameter of the first sleeve 231a, thereby ensuring the maximum injection molding space occupied by the tip 24 and facilitating the flow of injection liquid, whereby the wall thickness of each injection molding is even, thus greatly reducing the risk of bubble generation during injection molding.

Further, referring to FIG. 12, an anti-rotation groove 231a2 is further recessed in a surface of the first sleeve 231a. And/or an anti-rotation block 231b1 is provided on the second sleeve 231b. During injection molding of the tip 24, glue liquid flows into the anti-rotation groove 231a2 and encloses the anti-rotation block 231b1, whereby the tip 24 is fixed in an axial direction and a circumferential direction, and the connection strength and the limit strength are further ensured.

As shown in FIG. 7, FIG. 8, and FIG. 13, the tip 24 includes a first connecting section 241 and a guiding section 242 coaxially connected to a distal end of the first connecting section 241. The guiding section 242 is in the shape of a taper having an outer diameter gradually decreasing from a proximal end to a distal end thereof. A maximum outer diameter of the guiding section 242 is greater than an outer diameter of the first connecting section 241 located at a proximal end of the guiding section, whereby a joint of the guiding section 242 and the first connecting section 241 forms a step. A proximal face of the guiding section 242 abuts against a distal face of a sheath catheter when the sheath catheter is sleeved on the outer periphery of the sheath core 11. Preferably, the thickness of the step is approximately equal to the wall thickness of the sheath catheter, whereby an outer periphery of the sheath catheter may be flush with a maximum outer periphery of the tip 24 when the sheath catheter is sleeved on the outer periphery of the sheath core 11 and the distal face of the sheath catheter abuts against the proximal face of the guiding section 242, thus avoiding scratching a blood vessel wall during pushing.

In addition, referring to FIG. 7, since the inverted hook 23 is made of a polymer material slightly harder than that of the tip 24 and the hardness of the polymer material is much less than that of the metal material of the inverted buckle 22, a length L1' of the tapered extending section 233 at the front end of the inverted hook 23 is required to be not too large or small. If the length is too large, the overall bending and flexibility properties of the tip 24 are affected. If the length is too small, the taper changes greatly, the wall thickness of the tip 24 changes greatly, and injection molding bubbles may be generated. Preferably, an axial length L1' of the extending section 233 is less than or equal to one half of an axial length L2' of the guiding section. The axial length L1' of the extending section 233 crosses at least the step formed by the first connecting section and the guiding section of the tip. That is, the extending section 233 is at least partially embedded in the guiding section of the tip. This length avoids the generation of bubbles as much as possible, while ensuring the bending and flexibility properties of the tip 24.

In this embodiment, the sheath core assembly is provided with a long tapered extending section 233 adapted to the taper of the tip 24 at the distal end of the inverted hook 23, whereby the wall thickness of the tip 24 changes more evenly. At the same time, the smooth flow of injection molding glue liquid can be guided, thus effectively preventing the generation of injection molding bubbles. Also, this embodiment greatly simplifies the structure of the inverted buckle 22 since the connection mode of the inverted buckle 22 and the inverted hook 23 is changed, thereby ensuring the connection strength, reducing the difficulty of machining, and reducing the cost. Moreover, the inverted hook 23 is directly and integrally formed on the outer peripheries of the sheath core 21 and the inverted buckle 22 by injection molding, thereby omitting the mounting process of the inverted hook 23, simplifying the production process, and improving the production efficiency. In addition, the inverted hook 23 is provided with a clamping groove 231a1 in which the limiting arm 222 of the inverted buckle 22 is inserted, so as to increase the axial connection strength between the inverted hook 23 and the inverted buckle 22 and prevent the circumferential rotation of the inverted hook 23.

### Embodiment 3

This embodiment provides a method for manufacturing the sheath core assembly of Embodiment 2. Specifically, the manufacturing method includes the following steps.

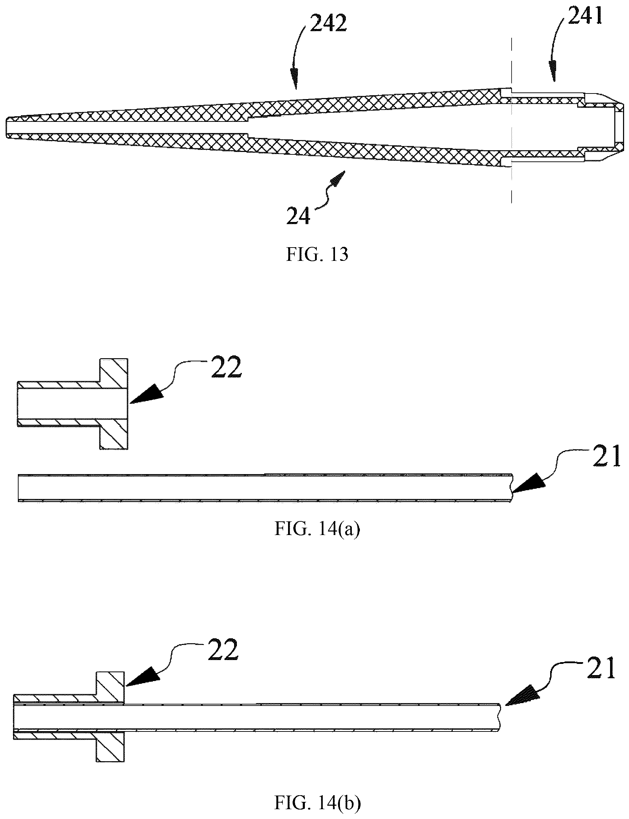

As shown in FIG. 14(a), a sheath core 21 and an inverted buckle 22 are provided. The sheath core 21 is a stainless steel tube, and the inverted buckle 22 is a machined metal member. Exemplarily, the inverted buckle 22 includes a second welding section 221 sleeved to the sheath core 21 and relatively fixed to the sheath core 21 and a limiting arm 222 radially extending from a proximal end of the second welding section 221.

As shown in FIG. 14(b), the inverted buckle 22 is coaxially sleeved to a distal end of the sheath core 21 and is relatively fixed to the sheath core 21. Specifically, the second welding section 221 of the inverted buckle 22 is welded to the sheath core 21, whereby the inverted buckle 22 and the sheath core 21 are relatively fixed.

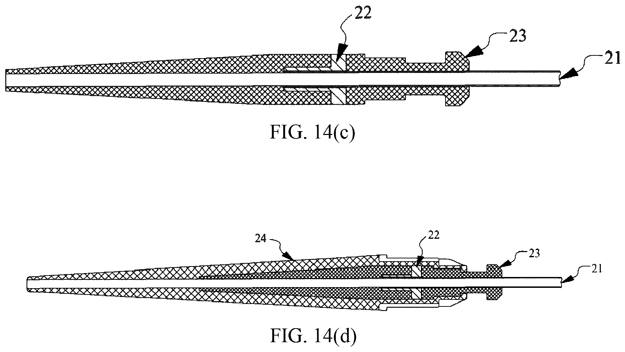

As shown in FIG. 14(c), an inverted hook 23 is formed at the distal end of the sheath core 21 and an outer periphery of the inverted buckle 22 by injection molding. The formed inverted buckle 23 includes a second connecting section 231 relatively fixed to the inverted buckle 22, and a blocking section 232 and an extending section 233 coaxially connected to a proximal end and a distal end of the second connecting section 231. The extending section 233 is tapered, and the extending section 233 has an outer diameter gradually decreasing from a proximal end to a distal end thereof. After injection molding, the inverted hook 23 wraps the distal end of the sheath core 21 and the outer periphery of the inverted buckle 22, and the limiting arm 222 of the inverted buckle 22 is embedded in the second connecting section 231 of the inverted hook 23, whereby a clamping groove 231a1 in which the limiting arm 222 is embedded is formed in the second connecting section 231. At this moment, the inverted buckle 22 and the inverted hook 23 are relatively fixed.

As shown in FIG. 14(d), a tip 24 is formed at the outer peripheries of the inverted buckle 22 and the second connecting section 231 by injection molding. The tip 24 includes a first connecting section and a guiding section coaxially connected to a distal end of the first connecting section. The guiding section is tapered, and the guiding section has an outer diameter gradually decreasing from a proximal end to a distal end thereof. A maximum outer diameter of the guiding section is greater than an outer diameter of the first connecting section located at the proximal end of the tip, whereby a joint of the guiding section and the first connecting section forms a step, and a proximal face of the guiding section abuts against a distal face of a sheath catheter when the sheath catheter is sleeved on an outer periphery of the sheath core 21. Preferably, a taper difference between the guiding section and the extending section 233 is ±5°. Preferably, the taper of the guiding section is equal to that of the extending section 233.

In the manufacturing method according to this embodiment, with the extending section 233 of the inverted hook 23, the wall thickness of each injection molding is even, thus greatly reducing the risk of bubbles generated by injection molding. In addition, the structure of the inverted buckle 22 is greatly simplified, the machining difficulty of the inverted buckle 22 is reduced, and the cost of parts is reduced. More importantly, through step-by-step integrated injection molding, the mounting process of the inverted hook 23 is omitted, thus simplifying the production process and improving the production efficiency.

### Embodiment 4

Based on the sheath core assemblies of Embodiments 1 and 2 described above, this embodiment provides a delivery device including the sheath core assembly of Embodiment 1 or Embodiment 2.

The present embodiment provides a sheath core assembly and a delivery device including the sheath core assembly. A distal end of a connector extends, and the shape of an extending section is adapted to a tapered guiding section of a tip as much as possible, whereby the wall thickness of the tip formed by injection molding is as even as possible, thus avoiding a large difference of the wall thickness. At the same time, the flow of injection liquid can be guided during injection molding, which is more beneficial to the molding of the tip and reduces the generation of cooling shrinkage bubbles during injection molding under the same technological conditions. In addition, the connector having the extending section may also improve the connection strength between the sheath core and the tip, and stability and reliability are taken into account. The present embodiment provides a method for manufacturing a sheath core assembly. The wall thickness of each injection molding is even, thus greatly reducing the risk of bubbles generated by injection molding. In addition, the structure of an inverted buckle is greatly simplified, the machining difficulty of the inverted buckle is reduced, and the cost of parts is reduced. More importantly, through step-by-step integrated injection molding, the mounting process of an inverted hook is omitted, thus simplifying the production process and improving the production efficiency.

The various technical features of the above embodiments may be combined randomly. In order to simplify the description, all possible combinations of the various technical features in the above-mentioned embodiments are not described. However, as long as the combinations of these technical features do not contradict, the combinations should be considered to be the scope of the description.

The above-mentioned embodiments express only a few implementations of the present disclosure, which are described in greater detail but are not to be construed as limiting the scope of the present disclosure. It will be appreciated by those of ordinary skill in the art that numerous variations and modifications may be made to the present disclosure without departing from the concept of the present disclosure, which fall within the protection scope of the present disclosure. Therefore, the protection scope of the present disclosure should be determined by the appended claims.

## Claims

1. A sheath core assembly, comprising a sheath core, and a tip and a connector which are provided at a distal end of the sheath core, wherein the tip comprises a first connecting section and a guiding section coaxially connected to a distal end of the first connecting section; the guiding section is tapered and has an outer diameter gradually decreasing from a proximal end to a distal end thereof; the connector is connected between the sheath core and the tip; a proximal end of the connector extends out from a proximal face of the tip, and a distal end of the connector extends towards a distal end of the tip and forms an extending section; the extending section is embedded in the guiding section; and the extending section is tapered and has an outer diameter gradually decreasing from a proximal end to a distal end thereof.

2. The sheath core assembly according to claim 1, wherein a taper difference between the guiding section and the extending section is ±5°.

3. The sheath core assembly according to claim 1 or 2, wherein the connector comprises an inverted buckle and an inverted hook; the inverted buckle is coaxially sleeved to the distal end of the sheath core and relatively fixed to the sheath core; the inverted hook comprises a second connecting section and a blocking section coaxially connected to a proximal end of the second connecting section, and the second connecting section is relatively fixed to the inverted buckle; and the tip wraps outer peripheries of the inverted buckle and the second connecting section, whereby only the blocking section extends out of the proximal face of the tip.

4. The sheath core assembly according to claim 3, wherein the inverted buckle comprises a third connecting section and the extending section coaxially connected to a distal end of the third connecting section; the third connecting section is relatively fixed to the sheath core after the inverted buckle is coaxially sleeved to the distal end of the sheath core; and the second connecting section is sleeved to the third connecting section and relatively fixed to the third connecting section after the inverted hook is sleeved to the distal end of the sheath core.

5. The sheath core assembly according to claim 4, wherein the third connecting section comprises a first welding section and a sleeving section coaxially connected to a proximal end of the first welding section; an outer diameter of the sleeving section is greater than that of the first welding section; an outer periphery of the sleeving section is provided with an external thread, and an inner periphery of the second connecting section is provided with an internal thread; the first welding section is relatively fixed to the sheath core after the inverted buckle is coaxially sleeved to the distal end of the sheath core; and the second connecting section is sleeved to the sleeving section and threaded to the sleeving section after the inverted hook is sleeved to the distal end of the sheath core.

6. The sheath core assembly according to claim 4, wherein an axial length of the extending section is less than or equal to one third of an axial length of the guiding section.

7. The sheath core assembly according to claim 4, wherein an anti-revolving groove is recessed in a surface of the extending section.

8. The sheath core assembly according to claim 3, wherein the inverted hook further comprises the extending section coaxially connected to a distal end of the second connecting section, and the inverted hook wraps the distal end of the sheath core and the outer periphery of the inverted buckle, whereby the inverted hook is relatively fixed to the inverted buckle.

9. The sheath core assembly according to claim 8, wherein the inverted buckle comprises a second welding section sleeved to the sheath core and relatively fixed to the sheath core and a limiting arm radially extending from a proximal end of the second welding section, and the limiting arm is embedded in the second connecting section of the inverted hook when the inverted hook wraps the distal end of the sheath core and the outer periphery of the inverted buckle, whereby the inverted hook is relatively fixed to the inverted buckle.

10. The sheath core assembly according to claim 9, wherein the second connecting section comprises a first sleeve and a second sleeve connected to each other; an outer diameter of the first sleeve is greater than that of the second sleeve; and the first sleeve is provided with a clamping groove in which the limiting arm is embedded.

11. The sheath core assembly according to claim 10, wherein at least two limiting arms are circumferentially provided at intervals at the proximal end of the second welding section; at least two clamping grooves are provided on the first sleeve; and one limiting arm is embedded in one clamping groove.

12. The sheath core assembly according to claim 9, wherein an anti-rotation groove is further recessed in a surface of the first sleeve, and/or an anti-rotation block is provided on the second sleeve.

13. The sheath core assembly according to claim 8, wherein an axial length of the extending section is less than or equal to one half of an axial length of the guiding section.

14. The sheath core assembly according to any one of claims 3 to 13, wherein the inverted buckle is a metal member, and/or the inverted hook is integrally formed by injection molding.

15. A method for manufacturing a sheath core assembly according to any one of claims 8 to 13, comprising:
providing a sheath core and an inverted buckle;
coaxially sleeving the inverted buckle to a distal end of the sheath core, and relatively fixing the inverted buckle to the sheath core;
forming an inverted hook at the distal end of the sheath core and an outer periphery of the inverted buckle by injection molding, wherein the formed inverted hook comprises a second connecting section relatively fixed to the inverted buckle, and a blocking section and an extending section coaxially connected to a proximal end and a distal end of the second connecting section, and the extending section is tapered and has an outer diameter gradually decreasing from a proximal end to a distal end thereof; and
then forming a tip at the outer peripheries of the inverted buckle and the second connecting section by injection molding, wherein the tip comprises a first connecting section and a guiding section coaxially connected to a distal end of the first connecting section, and the guiding section is tapered and has an outer diameter gradually decreasing from a proximal end to a distal end thereof.

16. A delivery device, comprising the sheath core assembly according to any one of claims 1 to 14.
